# EUROPEAN PATENT APPLICATION

(11) **EP 2 184 003 A1**
(43) Date of publication of application: **12.05.2010**
(21) Application number: 09252554.2
(22) Date of filing: 05.11.2009
(51) Int. Cl.: A61B 1/00, A61M 25/00

(54) **Endoscope and flexible tube therefor**

(30) Priority: 06.11.2008 JP 2008285005
(71) Applicant: FUJIFILM Corporation, Minato-ku Tokyo (JP)
(72) Inventor: Fukunaga, Toshiaki, Kanagawa (JP)
(74) Representative: Stevens, Jason Paul

(57) **Abstract**

A flexible tube (12A) for an endoscope is formed from a helical tube (32) formed with a strip-shaped member wound in a helical shape, and a mesh tube (34) at the outer periphery of the helical tube, the mesh tube having a ring-shaped cross-section and being formed from braided fine wires. A thermoplastic resin (35) is formed in a patterned shape at the outer periphery of the mesh tube, and an outer skin (36) is formed at the outer periphery of the mesh tube, over the thermoplastic resin, from a material that has a smaller thermal deformation than the thermoplastic resin. The flexible tube of the endoscope is not readily thermally deformed and is not readily imparted with a permanent or semi-permanent curvature during autoclave processing.

## Description

The present invention relates to an endoscope and a flexible tube therefor.

Medical endoscopes are used for inserting an insertion section thereof into a body cavity when observing internal organs and the like, and various types of treatment and procedures are performed by employing treatment instruments that are inserted into treatment instrument insertion channels of endoscopes. Consequently, when an endoscope that has been used once is to be reused on another patient, disinfection and sterilization of the endoscope must be performed after finishing the investigation or treatment in order to prevent contagion between patients via the endoscope. Disinfectant liquids, ethylene oxide gas, formalin gas, hydrogen peroxide gas plasma, ozone, or autoclaves that sterilize using high temperature and pressure steam are employed as methods for disinfection and sterilization.

Autoclaves that sterilize endoscopes with high temperature and pressure steam have already become widely used globally as a disinfection and sterilization method. This method has several merits, such as a highly reliable sterilization effect, no residual toxicity, cheap running cost, and the like. In American Standard ANSI/AAMI ST37-1992, issued by the Association for the Advancement of Medical Instrumentation and endorsed by the American National Standards Institute, typical conditions during high temperature and pressure steam sterilizing of endoscopes are four minutes at 132°C for a pre-vacuum sterilization process and 10 minutes at 132°C for a gravity sterilization process. However, there is the problem that great damage may be imparted to medical instrumentation under such conditions.

Flexible tubes for endoscopes are equipped with helical tubes formed from a strip-shaped member wound in a helical shape at a constant diameter, a mesh tube formed of fine wires braided into a ring shape around the outer periphery of the helical tube, and an external skin member that covers the outer periphery of the mesh tube. In particular, an endoscope flexible tube whose surface is covered by an external skin has been proposed (see Japanese Patent Application Laid-Open (JP-A) Nos. 2006-314521 and 2006-25843) as an endoscope flexible tube with excellent chemical liquid durability and autoclave durability, the external skin thereof being configured from a polyolefin thermoplastic elastomer.

However, in the flexible tube described in JP-A Nos. 2006-314521 and 2006-25843, a problem arises that the insertability of the flexible tube deteriorates due to the occurrence of thermal deformation when processing in an autoclave in a bent state.

In consideration of the above circumstances, at least the preferred embodiments of the present invention provide a flexible tube for an endoscope that is not readily imparted with a permanent or semi-permanent curvature even when it is autoclave processed.

According to a first aspect of the present invention, there is provided a flexible tube for an endoscope, the flexible tube including: a helical tube formed with a strip shaped member wound in a helical shape; a mesh tube, disposed at the outer periphery of the helical tube and formed in a ring shape from braided fine wires; a thermoplastic resin, disposed at the outer periphery of the mesh tube and formed in a patterned shape; and an outer skin disposed at the outer periphery of the mesh tube at which is formed the thermoplastic resin, the outer skin being formed from a material having a smaller thermal deformation than the thermoplastic resin.

According to the above configuration, the thermoplastic resin formed at the outer periphery of the mesh tube is formed in a patterned shape, and the outer skin at the outer periphery of the mesh tube at which is formed the thermoplastic resin is formed from a material having a smaller thermal deformation than the thermoplastic resin. As a result, the flexible tube of the endoscope is not readily thermally deformed and is not readily imparted with a permanent or semi-permanent curvature when processed in an autoclave.

The thermoplastic resin may be formed into plural ring shapes running in the circumferential direction of the mesh tube, or may be formed in a helical shape wound in the circumferential direction of the mesh tube.

By adopting such configurations, the thermoplastic resin is readily formed in a patterned shape since the thermoplastic resin is formed into plural ring shapes running in the circumferential direction of the mesh tube, or in a helical shape wound in the circumferential direction of the mesh tube.

The outer skin may be made from a fluoro-rubber or a silicone-rubber. By adopting such a configuration, the flexible tube of the endoscope is not readily thermally deformed and is not readily imparted with a permanent or semi-permanent curvature when processed in an autoclave, since the outer skin is made from a fluoro-rubber or from a silicone-rubber which have durability to high temperature and pressure steam (durability when autoclave processed).

At least the preferred embodiments of the flexible tube are not readily imparted with a permanent or semi-permanent curvature even when processed in an autoclave.

Preferred embodiments of the invention will now be described by way of example only and with reference to the accompanying drawings, in which:
FIG. 1 is a schematic configuration diagram of an endoscope with a flexible tube according to a first embodiment;
FIG. 2 is a partially cut-away side view illustrating the configuration of the flexible tube of the first embodiment;
FIG. 3 is a cross-section illustrating the configuration of the flexible tube of the first embodiment;
FIG. 4 is a partially cut-away perspective view illustrating the configuration of the flexible tube of the first embodiment;
FIG. 5 is a partially cut-away side view illustrating the configuration of a flexible tube for an endoscope according to a second embodiment, with thermoplastic resin formed into a helical shape;
FIG. 6 is a schematic diagram illustrating a method for forming the thermoplastic resin of the flexible tube according to the first embodiment; and
FIG. 7 is a schematic diagram illustrating another method for forming the thermoplastic resin of the flexible tube according to the first embodiment.

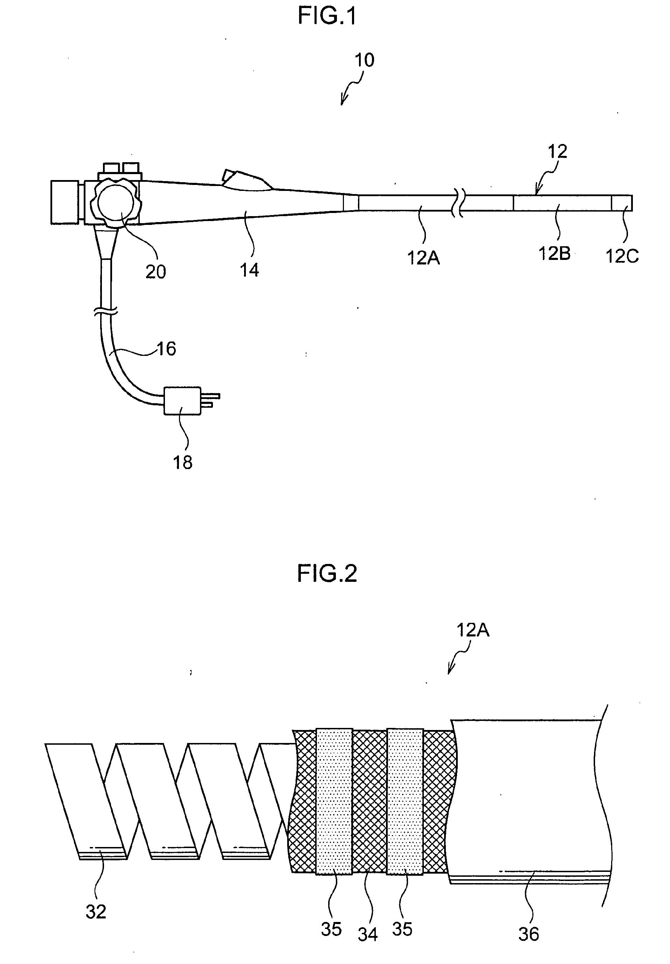

Explanation will first be given of the overall configuration of an endoscope 10, whose overall configuration is illustrated in FIG. 1.

The endoscope 10 shown in FIG. 1 is provided with an elongated shaped insertion section 12 for insertion into a body cavity of a patient, and a main body manipulation section 14 is connected to an end portion of the insertion section 12. An elongated shaped light guide flexible section 16 is connected to the main body manipulation section 14, the light guide flexible section 16 being detachably connectable to a light source device (not shown in the Figures). A connection section 18 equipped with terminal(s) for connection to the light source device is provided at the distal end of the light guide flexible section 16. A manipulation knob 20 for manipulating the insertion section 12 is provided on the main body manipulation section 14.

The insertion section 12 is equipped with: a flexible tube 12A, which makes up most of the length in the longitudinal direction (axial direction) of the insertion section 12, from a connection section thereof to the main body manipulation section 14; an angle section 12B connected to the distal end of the flexible tube 12A; and a distal end portion main body 12C connected to the distal end of the angle section 12B and internally installed with an object optical system and the like.

The angle section 12B is configured so as to remotely bendable by rotational manipulation of the manipulation knob 20 provided on the main body manipulation section 14. The light guide flexible section 16 is also of substantially the same structure as the flexible tube 12A of the insertion section 12.

The flexible tube 12A is set with a length that ensures the distal end portion main body 12C can reach the inside of a specific portion subject to observation, and is of a length such that an operator can grip the main body manipulation section 14 and operate away from the patient or the like without difficulty. The flexible tube 12A needs to have some flexibility over substantially the entire length thereof, with locations thereof that are inserted into the body cavity or the like of a patient being of a structure that is particularly more flexible.

The flexible tube 12A, particularly at the portion thereof connected to the main body manipulation section 14, needs to have a certain rigidity to bending (flexural rigidity) in order to obtain insertion propulsion force when inserting into a body cavity or the like. In particular the flexible tube 12A preferably has greater flexibility at the portion thereof connected to the angle section 12B in order to be able to substantially follow the bent shape of the angle section 12B when the angle section 12B is bent.

While not illustrated in the Figures, the flexible tube 12A of the endoscope is internally mounted with a light guide, image guide (signal cable in the case of an electronic endoscope), treatment instrument insertion channel, gas and liquid transmission tube, and the like.

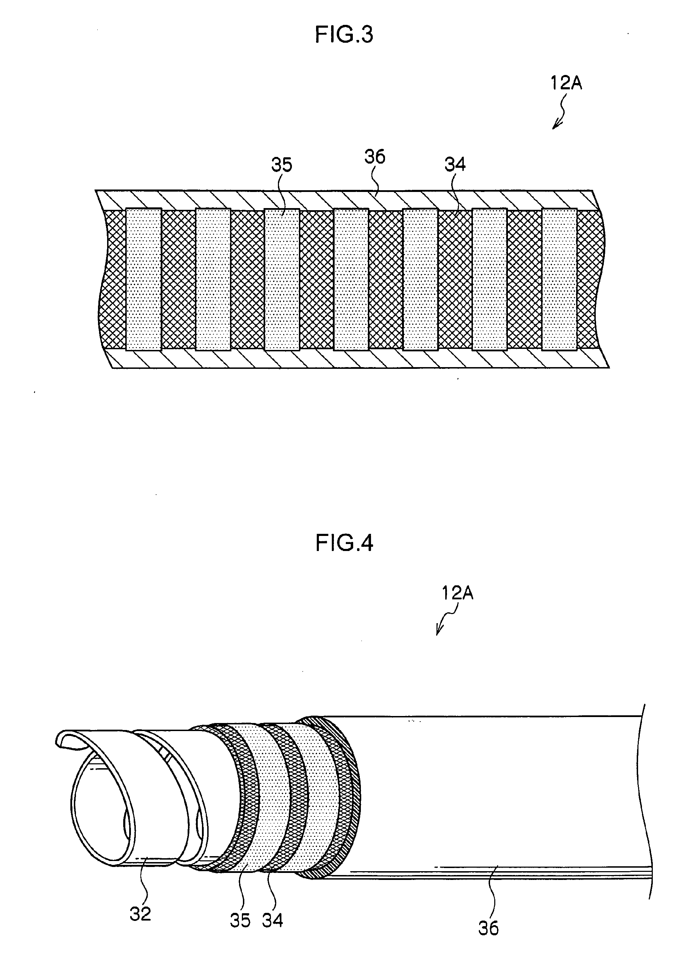

A partially cut-away side view of the flexible tube 12A is illustrated in FIG. 2. A cross-section of the flexible tube 12A is illustrated in FIG. 3, and a partially cut-away perspective view of the flexible tube 12A is illustrated in FIG. 4.

As shown in these figures, the flexible tube 12A is equipped with: a helical tube 32 shaped (formed) from a metal strip-shaped member wound into a helical shape; a mesh tube 34, shaped such that its cross-section is ring-shaped and formed of braided fine wires (wire material) made from a metal, at the outer periphery of the helical tube 32; a thermoplastic resin 35 formed at the outer periphery of the mesh tube 34; and an external skin 36 formed at the outer periphery of the mesh tube 34 at which is formed the thermoplastic resin 35.

The external skin 36 is formed from a material that has a thermal deformation (residual deformation when returned to room temperature) at least less than that of the thermoplastic resin 35, and, for example, is formed from a fluoro-rubber or silicone-rubber material.

A fluoro-rubber or silicone-rubber has durability to high temperature and pressure steam (durability when autoclave processed); however, the bending rigidity is less than that of the thermoplastic resin 35.

The thermoplastic resin 35 is directly formed with a patterned shape at the outer periphery of the mesh tube 34. The patterned shape is such that there are plural portions on the outer periphery of the mesh tube 34 formed with the thermoplastic resin 35, and plural portions thereon not formed with the thermoplastic resin 35.

In the present embodiment, specifically, the patterned shape is not linearly continuous in the longitudinal direction of the flexible tube 12A, namely, the thermoplastic resin 35 is intermittently present when viewed in a straight line along the longitudinal direction of the flexible tube 12A, with portions formed with the thermoplastic resin 35 and portions not formed with the thermoplastic resin 35.

More specifically, as shown in FIGS. 2, 3, and 4, patterned shape of the thermoplastic resin 35 is in plural ring shapes formed separated at specific intervals and running in the circumferential direction of the mesh tube 34.

Also, the thermoplastic resin 35 functions as an adhesive for joining the external skin 36 to the mesh tube 34. The thermoplastic resin 35 has conjugative ability to each of the mesh tube 34 and the external skin 36 that is at least higher than the conjugative ability of the external skin 36 to the mesh tube 34, thereby raising the conjugative ability between the external skin 36 and the mesh tube 34.

Other elastomers, such as, for example, a polyamide elastomer, a fluoro-elastomer, a styrene elastomer, a polyester elastomer, or a polyurethane elastomer, may be employed as the thermoplastic resin 35 in place of an olefin elastomer, such as a polypropylene resin, an ethylene-propylene copolymer resin, or the like.

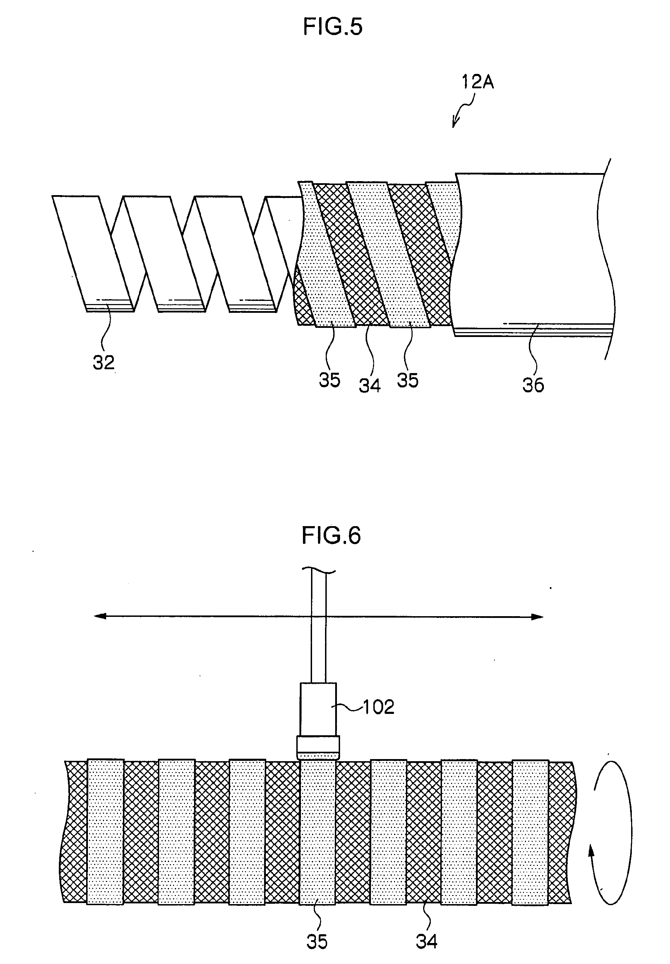

It should be noted that the thermoplastic resin 35 is not limited to being formed with the ring shaped patterned shape as illustrated in FIGS. 2, 3, and 4, as long as a patterned shape is formed so as to partially cover the peripheral face of the mesh tube. As the patterned shape various shapes may be envisaged, such as a striped shape, a zigzag shape, or the like; for example, a spiral shape may be made, as shown in FIG. 5, wound in the circumferential direction of the mesh tube 34.

Explanation will now be given of an example of a forming method of the external skin 36.

First, the thermoplastic resin 35 is applied to the outer periphery of the mesh tube 34. Specifically, as shown in FIG. 6, the mesh tube 34 is rotated in the circumferential direction thereof by a rotation unit, and after rotating the mesh tube 34 and applying a silane coupling agent thereto, an extrusion device 102 is moved along the longitudinal direction of the mesh tube 34, and the thermoplastic resin 35 is extruded from a nozzle in the extrusion device 102 and coated. Rotating the mesh tube 34 enables a patterned shape to be formed in a ring shape, a helical shape, or a stripe shape. The temperature of the thermoplastic resin 35 when so doing exceeds the melting point thereof.

The coating width and thickness of the thermoplastic resin 35 can be controlled by varying the shape of the nozzle of the extrusion device 102. The cover ratio of the thermoplastic resin 35 can be controlled for example by the extrusion pressure.

In order to suppress thermal deformation of the flexible tube 12A the cover ratio of the surface of the mesh tube 34 is preferably 80% or less. In order to ensure adhesive strength to the external skin 36 the cover ratio of the surface of the mesh tube 34 is preferably 20% or greater.

From the standpoint of suppressing thermal deformation, the coating width is preferably about 2 to 3 mm, and from the standpoint of suppressing undulations the coating thickness is preferably about 0.1 mm or less.

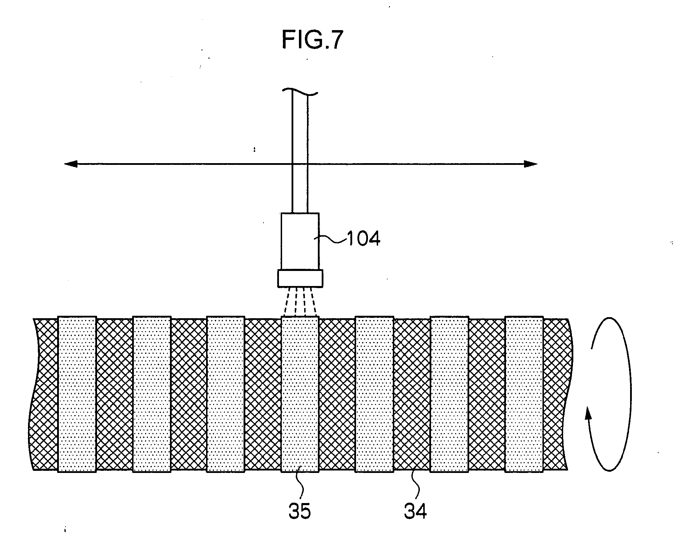

It should be noted that the configuration for applying the thermoplastic resin 35 is not limited to a method using the extrusion device 102, and various methods may be employed. For example, as shown in FIG. 7, thermoplastic resin 35 dissolved in a solvent may be ejected and applied from an ejection head 104.

The external skin 36 made from a fluoro-rubber is then formed onto the outer periphery of the mesh tube 34 at which is formed the thermoplastic resin 35. The external skin 36 is vulcanize bonded at high temperature onto both the mesh tube 34 and the thermoplastic resin 35 using a primer.

According to the flexible tube 12A of the endoscope 10 of the embodiments described above, the thermoplastic resin 35 formed onto the outer periphery of the mesh tube 34 is in a patterned shape, and also the external skin 36 formed onto the outer periphery of the mesh tube 34 at which is formed the thermoplastic resin 35 is made from a material having a smaller thermal deformation than the thermoplastic resin 35.

Consequently, the flexible tube 12A of the endoscope 10 is not readily thermally deformed and not readily imparted with a permanent or semi-permanent curvature when autoclave processing.

Since the external skin 36 is fusion bonded to the mesh tube 34 through the thermoplastic resin 35 without employing an adhesive, the external skin 36 does not readily separate from the mesh tube 34 even when subjected to repeated autoclave processing.

The rigidity to bending (flexural rigidity) of the flexible tube 12A can be adjusted to a specific value by varying the density and pitch of the pattern of the thermoplastic resin 35. The rigidity to bending of the flexible tube 12A can be adjusted to specific values at locations in the longitudinal direction thereof by varying the density and pitch of the pattern of the thermoplastic resin 35 at locations in the longitudinal direction (axial direction). For example, at locations of the flexible tube 12A that are near to the main body manipulation section 14, the rigidity to bending can be raised in order to obtain insertion propulsion force when inserting into a body cavity or the like. In addition, for example, the rigidity to bending of the portion of the flexible tube 12A near to the angle section 12B can be made lower than that of the portions near to the main body manipulation section 14 in order to be able to substantially follow the bent shape of the angle section 12B when the angle section 12B is bent.

The present invention is not limited to the above embodiments, and various changes, modifications and improvements are possible thereto.

## Claims

1. A flexible tube (12A) for an endoscope (10), the flexible tube comprising:
a helical tube (32) formed from a strip-shaped member wound in a helical shape;
a mesh tube (34), disposed at the outer periphery of the helical tube (32) and formed from braided fine wires such that cross-section of the mesh tube (34) is ring-shaped;
a thermoplastic resin (35), disposed at the outer periphery of the mesh tube (34) and formed in a patterned shape; and
an outer skin (36) disposed at the outer periphery of the mesh tube (34) at which is formed the thermoplastic resin (35), the outer skin (36) being formed from a material having a smaller thermal deformation than the thermoplastic resin (35).

2. The flexible tube of claim 1, wherein the thermoplastic resin (35) is formed into a plurality of ring shapes running in the circumferential direction of the mesh tube (34).

3. The flexible tube of claim 1, wherein the thermoplastic resin (35) is formed in a helical shape wound in the circumferential direction of the mesh tube (34).

4. The flexible tube of any preceding claim, wherein the outer skin (36) is made from a fluoro-rubber or from a silicone-rubber.

5. The flexible tube of any preceding claim, wherein the cover ratio of the thermoplastic resin (35) on the surface of the mesh tube (34) is from 20% to 80%.

6. The flexible tube of claim 1, wherein the thermoplastic resin (35) is formed in a patterned shape of covering portions repeating along the longitudinal direction of the mesh tube (34) that partially cover the surface of the mesh tube (34).

7. An endoscope (10) comprising:
an elongated insertion section (12) that is inserted into a body cavity of a patient;
a manipulation section (14) connected to the proximal end of the insertion portion (12); and
an elongated light guide flexible section (16) connected to the manipulation portion (14), the distal end of the light guide flexible section (16) provided with a terminal (18) detachably connectable to a light source device,
the insertion section (12) including a flexible tube (12A) which makes up most of the length, in the longitudinal direction, of the insertion section (12) from a section thereof connected to the manipulation section (14), wherein the flexible tube (12A) comprises a flexible tube (12A) as claimed in any preceding claim.
